# EUROPÄISCHE PATENTANMELDUNG

(11) **EP 2 543 412 A1**
(43) Veröffentlichungstag der Anmeldung: **09.01.2013**
(21) Anmeldenummer: 11172721.0
(22) Anmeldetag: 05.07.2011
(51) Int. Cl.: A61N 5/10

(54) **Strahlerweichenmodul für einen Afterloader sowie Afterloadersystem mit Afterloader und Strahlerweichenmodul**

(71) Anmelder: Eckert & Ziegler Bebig GmbH, 13125 Berlin (DE)
(72) Erfinder: Kühlborn, Bernd, 38120 Braunschweig (DE); Wruck, Stefan, 13189 Berlin (DE)
(74) Vertreter: Gulde Hengelhaupt Ziebig & Schneider

(57) **Zusammenfassung**

Die Erfindung betrifft ein modulares Strahlerweichenmodul (1) für einen Brachytherapie-Afterloader umfassend eine Aufnahme (8) für eine Transferverbindung (7) eines Strahlers/Dummies (10), ein Mittel (6a) zur Referenzierung der Position eines Strahlers/Dummies (10), und ein Verteilermodul (4) umfassend eine Eingangsseite (4a) mit einer Mehrzahl von ersten Kanälen (11) und eine Ausgangsseite (4b) mit einer Mehrzahl von zweiten Kanälen (12), wobei jeder erste und jeder zweite Kanal (11, 12) zur Aufnahme eines Strahlers/Dummies (10) ausgebildet ist. Ferner ist eine Verstelleinheit (3) zur Positionierung eines Strahlers/Dummies (10) vor einem ersten Kanal (11) des Verteilermoduls (4) vorgesehen sowie ein Führungskanal (9) zur Führung eines Strahlers/Dummies (10) von der Aufnahme (8) zur Verstelleinheit (3), und ein Mittel (5) zum Anschluss von Applikationen verbunden mit der Ausgangsseite des Verteilermoduls (4). Ferner ist ein Brachytherapie-Afterloadersystem bestehend aus Afterloader (20) und modularem Strahlerweichenmodul (1) vorgesehen.

## Beschreibung

Die vorliegende Erfindung betrifft ein Strahlerweichenmodul für einen Afterloader sowie ein Afterloadersystem mit einem Afterloader und dem erfindungsgemäßen Strahlerweichenmodul. Das Strahlerweichenmodul ist insbesondere von einem Gehäuse des Afterloaders trennbar oder getrennt ausgebildet und mit diesem über eine Transferverbindung verbindbar.

### Stand der Technik

Zur Behandlung von Krebs und anderer Krankheiten wird häufig Strahlung eingesetzt. Die Brachytherapie ist eine Form der Strahlentherapie, bei der eine radioaktive Strahlenquelle innerhalb oder in unmittelbarer Nähe des zu bestrahlenden Gebietes im Körper platziert wird. Im Gegensatz zu einer externen Strahlentherapie bzw. einer Bestrahlung von außen, bei der hochenergetische Strahlen von außerhalb des Körpers auf den Tumor gerichtet werden, bedeutet die Brachytherapie, dass Strahlenquellen direkt am Ort des Krebsgeschwulstes platziert werden.

Vorhandene Geräte für die Brachytherapie, sogenannte Nachlader oder Afterloader, arbeiten generell nach einem einheitlichen Schema. Dabei wird eine Applikation über lange Transferschläuche mit dem Gerät verbunden. Dieses Prinzip bedingt eine Reihe von Fehlerquellen für die Positionierung des radioaktiven Strahlers und auch für den Anschluss der Applikation.

Für die Positionierung des Strahlers sind in der Norm DIN EN 60601-2-17 konkrete Anforderungen formuliert. Die Positionierung muss mit einer Genauigkeit von +/- 2mm erfolgen. Um diese Anforderung zu erfüllen, arbeiten Afterloadersysteme mit zwei grundlegend verschiedenen Prinzipien. Eine Möglichkeit besteht darin mit fixen Systemlängen zu arbeiten. Dieser Ansatz bedingt hohe Ansprüche an die eingesetzten Transferschläuche, und Applikatoren. Mehrere Applikatoren zum Applizieren von radioaktiven Strahlern können dabei eine Applikation ausbilden. Längenänderungen führen hier zwangsläufig zu Abweichungen bei der Positionierung. Eine weitere Möglichkeit besteht darin, mit Hilfe einer nicht radioaktiven Attrappe, auch Dummy genannt, eine Messung der (angekoppelten) Systemlänge der Applikation durchzuführen. Auf der Basis dieser Messung kann dann die Positionierung des Strahlers vorgenommen werden. Längenänderungen von Transferschläuchen werden bei der Positionierung also berücksichtigt. Verschiedene Faktoren haben aber bei beiden aufgezeigten Prinzipien einen negativen Einfluss auf die Güte der Positionierung des Strahlers. Negativ wirken sich hier verschiedene Innendurchmesser der Transferschläuche, die Verlegung der Transferschläuche in Radien sowie ggf. die bereits erwähnten Längenänderungen der Transferschläuche aus. Es wird somit durch einen Dummy zunächst die Länge des Systems vermessen und überprüft, bevor die Strahler appliziert werden.

Der Vortrieb von Dummy und Strahler erfolgt bei beiden Prinzipien über die Friktion zwischen einem Riemen und einer Seilscheibe. Hier kommt es zwangsläufig zu geringen Abweichungen. Diese Abweichungen summieren sich ab Passieren eines Referenzpunkts auf. Bei beiden Prinzipien kann eine Referenzierung von Dummy bzw. Strahler nur innerhalb des Gerätes erfolgen.

Der Anschluss von Applikationen mit langen Transferschläuchen bedingt auch die entsprechenden Transitzeiten für den Strahler. Der Strahler muss für jeden an die Strahlerweiche angeschlossenen Kanal die gesamte Strecke in das Gerät zurückfahren und dann, nach Verstellung der Strahlerweiche, wieder ausfahren.

Diese Transitzeiten führen zu einer zusätzlichen Strahlenbelastung des Patienten.

Der Anschluss von Applikationen mit langen Transferschläuchen bedingt auch entsprechende Transitzeiten für den Dummy. Der Dummy muss für jeden angeschlossenen Kanal die gesamte Strecke in das Gerät zurückfahren und dann, nach Verstellung der Strahlerweiche, wieder ausfahren. Diese Transitzeiten führen zu einer Verlängerung der Behandlungszeiten.

Der Anschluss von Applikationen mit langen Transferschläuchen bedingt weiterhin eine gewisse Gefahr der Verwechslung.

Der Anschluss von bestimmten Applikationen mit flexiblen Kathetern oder mit Nadeln bedingt den Anschluss einer Vielzahl von Transferschläuchen.

Die Empfindlichkeit des Systems gegen durchhängende Transferschläuche macht zudem eine Ausrichtung von Patient und Gerät erforderlich. Das durch die Abschirmung schwere Gerät muss durch das Personal verfahren werden.

### Offenbarung der Erfindung

Das erfindungsgemäße Strahlerweichenmodul und das erfindungsgemäße Afterloadersystem sollen die Nachteile des Standes der Technik überwinden.

Es wird daher erfindungsgemäß ein Strahlerweichenmodul für einen Brachytherapie-Afterloader vorgeschlagen, umfassend eine Aufnahme für eine Transferverbindung eines Strahlers/Dummies, ein Mittel zur Referenzierung der Position eines Strahlers/Dummies, ein Verteilermodul umfassend eine Mehrzahl von ersten Kanälen, wobei jeder erste zur Aufnahme eines Strahlers/Dummies ausgebildet ist, eine Verstelleinheit zur Positionierung eines Strahlers/Dummies vor einem ersten Kanal des Verteilermoduls, einen Führungskanal zur Führung eines Strahlers/Dummies von der Aufnahme zur Verstelleinheit, und
ein Mittel zum Anschluss von Applikationen und/oder Applikatoren umfassend eine Mehrzahl von zweiten Kanälen, verbunden mit der Ausgangsseite des Verteilermoduls.

Das erfindungsgemäße Strahlerweichenmodul erlaubt die räumliche Trennung von Strahlerweiche und Afterloader durch den Anschluss an eine Transferverbindung sowie die in der Weiche angeordneten Referenzierungs- und/oder Messmittel.

Der Dummy bzw. Strahler wird in dem Strahlerweichenmodul selbst referenziert. Somit können Abweichungen nur noch auf dem verbleibenden Rest der Fahrstrecke entstehen und reduzieren sich damit in der Größenordnung von ca. 60%. Die Positionierung des Strahlers wird robuster.

Ferner wird die Wegstrecke zwischen Strahlerweichenmodul und Applikation deutlich reduziert. Der Strahler/Dummy muss nicht mehr für jeden angeschlossenen Kanal die komplette Wegstrecke zwischen Afterloader und Applikation abfahren, sondern fährt für einen neuen Kanal nur bis hinter die Verstelleinheit des Strahlerweichenmoduls zurück. Nach einer schnellen Verstellung der Strahlerweiche ändert der Strahler dann direkt die Fahrtrichtung und fährt in den neuen Kanal ein. Es kommt zu einer deutlichen Reduzierung der Transitzeiten und somit zu einer Reduzierung der Strahlenbelastung.

Durch die Verlagerung der Anschlüsse für die Ankopplung von Transferschläuchen oder auch direkt von Applikationen, z. B. flexiblen Katheter oder Nadeln, in den Bereich der Anwendung, wird die Gefahr von Verwechslungen reduziert.

Der Aufwand für den Anschluss der Applikation wird reduziert. Die Applikationen könnten direkt oder über Adapter an dem Strahlerweichenmodul angekoppelt werden.

Eine Ausrichtung von Patient und Gerät ist nicht mehr erforderlich. Das System kann die entstehenden Fehler kompensieren. Ein Verschieben des schweren Afterloaders ist nicht mehr erforderlich.

Die Strahlerweiche kann so ausgebildet sein, dass sie zur selben Zeit sowohl einen Strahler wie auch einen Dummy aufnehmen kann. Bevorzugt kann sie nur einen Strahler oder nur einen Dummy aufnehmen und Dummy bzw. Strahler werden zeitlich sequentiell aufgenommen.

Bevorzugt umfasst das Strahlerweichenmodul ferner ein Mittel zur Messung des Vortriebs von Strahler/Dummy.

Ferner kann auch eine Antriebseinheit zum Betrieb der Verstelleinheit sowie einen Anschluss an eine externe Energieversorgung vorgesehen sein. Die Antriebseinheit erlaubt die gesteuerte Auswahl von Kanälen zum Transport eines Strahlers oder eines Dummies in eine Applikation bzw. in einen Applikator.

Der Anschluss an eine externe Energieversorgung kann über die Aufnahme für eine Transferverbindung bzw. die Transferverbindung selbst erfolgen.

Es kann aber auch eine Energieversorgung in der Strahlerweiche selbst, z. B. in Form einer Batterie vorgesehen sein.

Der Führungskanal kann bevorzugt durch die Transferverbindung selbst ausgebildet sein, die mit der Verstelleinheit verbunden ist und über die Aufnahme in das Strahlerweichenmodul eingeführt wird.

Das Strahlerweichenmodul kann auch ein Mittel zur kabellosen Übertragung von Signalen an einen Afterloader aufweisen. Dann entfällt die Signalleitung in der Transferverbindung.

Durch die Ausbildung des Anschlusses muss lediglich eine einzige Verbindungsleitung mit dem Strahlerweichenmodul verbunden werden. An dem Afterloader muss also nicht mehr eine Vielzahl von Transferschläuchen angeordnet werden.

Das Mittel zum Anschluss von Applikationen ist bevorzugt austauschbar ausgebildet. Dies ermöglicht vorteilhaft die Veränderung des Strahlerweichenmoduls auf eine bestimmte Anzahl und Größe von Applikationen hin.

Die ersten Kanäle und die zweiten Kanäle sind bevorzugt derart ausgebildet, dass zu jedem zweiten Kanal mindestens ein erster Kanal vorhanden ist.

Dies stellt sicher, dass auch jeder zweite Kanal tatsächlich von einem ersten Kanal bedient werden kann. Erster und zweiter Kanal sind dabei so angeordnet, dass je ein zweiter Kanal in einen ersten Kanal übergehend verbunden ist.

Das Verteilermodul kann einen von der Eingangsseite her wachsenden oder gleichen Querschnitt aufweisen. Im Verteilermodul können die Abstände zwischen benachbarten ersten Kanälen an der Eingangsseite kleiner oder gleich den Abständen an der Ausgangsseite sein. Dadurch können die ersten Kanäle aufgefächert werden und die Anbringung von Applikationen an der Anschlussplatte erleichtert werden.

Weiter wird erfindungsgemäß ein Brachytherapie-Afterloadersystem vorgeschlagen, umfassend einen Afterloader, ein erfindungsgemäßes Strahlerweichenmodul und eine Transferverbindung zur Verbindung von Strahlerweichenmodul und Afterloader. Der Afterloader umfasst dabei ein Gehäuse, beinhaltet Strahler und Dummies, mindestens ein Mittel zum Vortrieb von Strahler und/oder Dummy, eine erste Abschirmung zur Strahlungsabschirmung, sowie eine Austrittsöffnung für Strahler und/oder Dummy. Die Transferverbindung zur Verbindung von Afterloader und Strahlerweichenmodul ist dabei so ausgebildet, dass sie mindestens einen Strahler oder einen Dummy führen kann.

Die Aufteilung des Systems in Afterloader, Strahlerweichenmodul und Transferverbindung erlaubt die räumliche Trennung von Strahlerweichenmodul und Afterloader und die Verkürzung der Distanz zwischen Strahlerweichenmodul und Applikation.

Das Strahlerweichenmodul ist bevorzugt vom Gehäuse des Afterloaders räumlich getrennt ausgebildet, bevorzugt mit einer Distanz von mehr als 30cm, noch bevorzugter mehr als 50cmm, und mit dem Afterloader über die Transferverbindung, bevorzugt genau eine Transferverbindung, verbindbar.

Die Transferverbindung ist bevorzugt über die Austrittsöffnung für Strahler und/oder Dummy mit dem Afterloader verbunden und ist über die Aufnahme für die Transferverbindung mit dem Strahlerweichenmodul verbunden.

Die Transferverbindung kann eine Energieversorgungsleitung umfassen. Sie kann ferner Signalleitungen und/oder eine zweite Abschirmung zur Strahlungsabschirmung umfassen.

Die Austrittsöffnung kann sowohl eine Austrittsöffnung für einen Strahler wie auch eine Austrittsöffnung für einen Dummy umfassen.

In einem Ausführungsbeispiel kann ein Schwenkarm am Gehäuse des Afterloaders angeordnet sein. Das Strahlerweichenmodul ist dann am Schwenkarm befestigt oder bevorzugt befestigbar und abtrennbar.

Der Schwenkarm ermöglicht ein besonders einfaches Verbringen von Strahlerweichenmodul zur Applikation.

Ferner kann das System einen Mikroprozessor umfassen, der das Mittel zum Vortrieb von Strahler und/oder Dummy steuert und der über die Signalleitungen in der Transferverbindung oder eine kabellose Übertragung Daten des Mittels zur Referenzierung und der Messung des Vortriebs aus dem Strahlerweichenmodul erhält.

Somit können sowohl Afterloader als auch Strahlerweichenmodul gesteuert oder überwacht werden.

Ein Strahler ist dabei bevorzugt ein Draht, der an seinem distalen Ende eine Strahlungsquelle aufweist, bevorzugt eine Ir-192 Strahlungsquelle oder ein Co-60 Strahlungsquelle. Ein Dummy weist keine Strahlungsquelle auf, entspricht aber in seinen Größenabmessungen dem Strahler.

Vorteilhafte Weiterbildungen der Erfindung sind in der Beschreibung beschrieben.

### Zeichnungen

Ausführungsbeispiele der Erfindung werden anhand der Zeichnungen und der nachfolgenden Beschreibung näher erläutert. Es zeigen:
- Figur 1: eine Afterloader des Standes der Technik
- Figur 2: ein erfindungsgemäßes Strahlerweichenmodul,
- Figur 3: Einlass- und Auslassseite eines erfindungsgemäßen Verteilermoduls in einem Ausführungsbeispiel, und
- Figur 4: ein erfindungsgemäßes Afterloadersystem in einem ersten Ausfüh-rungsbeispiel,
- Figur 5: ein erfindungsgemäßes Afterloadersystem in einem zweiten Ausfüh-rungsbeispiel.

### Ausführungsformen der Erfindung

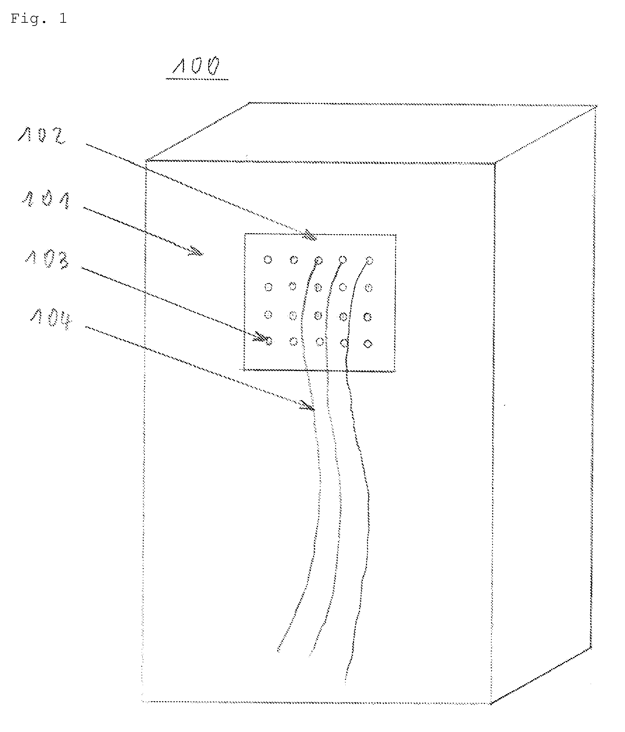

In der Figur 1 ist eine Afterloader 100 des Standes der Technik gezeigt. Das Gerät umfasst ein Gehäuse 101 und eine im Gehäuse 101 integrierte Strahlerweiche 102. Die Strahlerweiche 102 umfasst eine Mehrzahl von Kanälen 103 zum Anschluss von Transferschläuchen 104. Das Gerät 100 ist aufgrund seiner notwendigen Strahlungsabschirmung sehr schwer und für den Anwender schwierig zu bewegen.

Der erfindungsgemäße Ansatz erlaubt die fixe Verbindung von Afterloader 100 und Strahlerweiche 102 aufzutrennen, die Strahlerweiche 102 wird zu einer eigenständigen Einheit. In der Folge kann die Strahlerweiche 102 direkt im Bereich einer Applikation platziert werden.

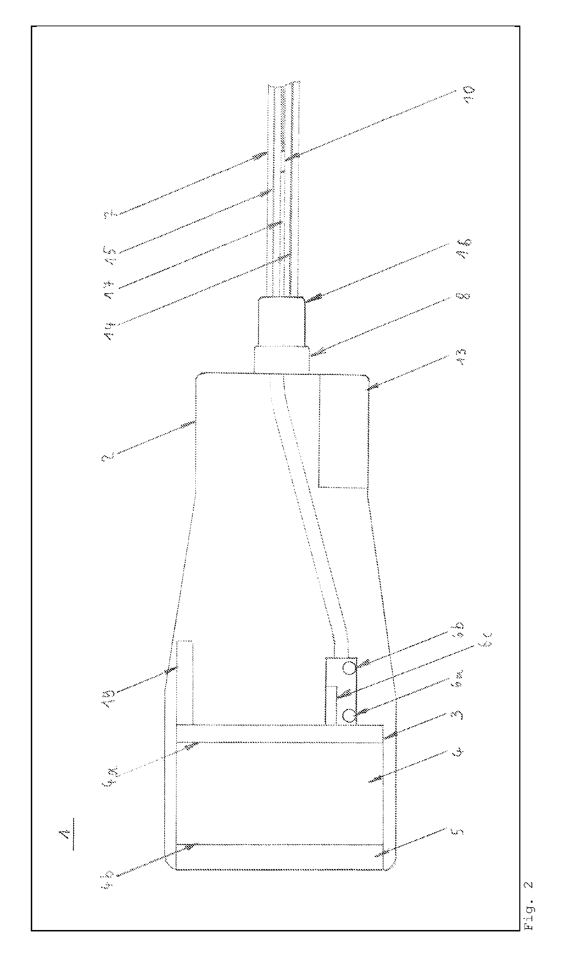

Das erfindungsgemäße Strahlerweichenmodul 1 ist in Fig. 2 gezeigt. Das Strahlerweichenmodul 1 ist von dem Afterloader getrennt oder trennbar ausgebildet. Es umfasst ein Gehäuse 2, in dem sich die weiteren Elemente befinden. So ist im Gehäuse 2 zunächst eine Aufnahme 8 für eine Transferverbindung 7 vorgesehen, über die ein Strahler oder ein Dummy oder sowohl ein Strahler als auch ein Dummy in das Strahlerweichenmodul 1 einführbar ist/sind. Bevorzugt wird an die Aufnahme 8 eine Transferverbindung 7 angeschlossen, die im Inneren eine Führung für Strahler und/oder Dummy beinhaltet. Die Transferverbindung 7 kann mit einer Abschirmung 16 ausgestattet sein, damit die Umgebung vor einer Strahlenbelastung geschützt wird.

Die Transferverbindung 7 stellt die Verbindung mit einem Afterloader 20 her. Die Transferverbindung 7 beinhaltet mindestens einen Fahrkanal 17 für Dummy und/oder Strahler 10, bevorzugt genau einen Fahrkanal 17, kann aber auch einen Fahrkanal 17 für einen Strahler 10 sowie einen Fahrkanal 17 für einen Dummy 10 enthalten. Ferner können in der Transferverbindung 7 optional ein Signalleitung 15 und eine Energieversorgungsleitung 14 für die Sensoren 6 und eine optionale Antriebseinheit 19 angeordnet sein. Jedoch kann im Strahlerweichenmodul 1 auch eine Energieversorgungseinheit 13 direkt integriert sein, z. B. in Form einer wiederaufladbaren Batterie. Ferner kann im Strahlerweichenmodul 1 auch ein Mittel zur kabellosen Übertragung von Signalen angeordnet sein. Dann kann die Signalleitung in der Transferverbindung 7 entfallen.

Über die Transferverbindung 7 wird ein Strahler und/oder ein Dummy 10 in das Strahlerweichenmodul 1 eingeführt. Von dort aus wird der Strahler und/oder der Dummy über einen Führungskanal 9 an einem Mittel 6a zur Referenzierung der Position des Strahlers vorbeigeführt. Ein solches Mittel 6a kann ein optischer Sensor sein. Ferner kann rein optional zusätzlich der Strahler/Dummy 10 an einem Mittel 6b zur Messung des Vortriebs von Strahler/Dummy 10 vorbeigeführt werden. Es ist bevorzugt ein Führungsrad kombiniert mit einem Messrad. Hier erfolgen die Referenzierung sowie optional die Messung des Vortriebs von Dummy bzw. Strahler 10. Durch die Verlagerung des Messsystems in das Strahlerweichenmodul 1 kann ein Fehler nur noch im Bereich der Applikation verursacht werden. Das führt zu einer Reduzierung von möglichen Positionsfehlern.

Strahler und/oder Dummy 10 sind dann mit einer Verstelleinheit 3 über den Führungskanal 9 verbunden. Der Führungskanal 9 kann auch durch die Transferverbindung 7 selbst ausgebildet sein. Dann wird der Führungskanal 7 über die Aufnahme 8 in das Strahlerweichenmodul 1 eingeführt und im Inneren mit der Verstelleinheit 3 verbunden. Dies kann dadurch geschehen, dass sich das Strahlerweichenmodul 1 zunächst in einem geöffneten Zustand befindet und nach Befestigung des Führungskanals 7 an der Verstelleinheit 3 geschlossen wird.

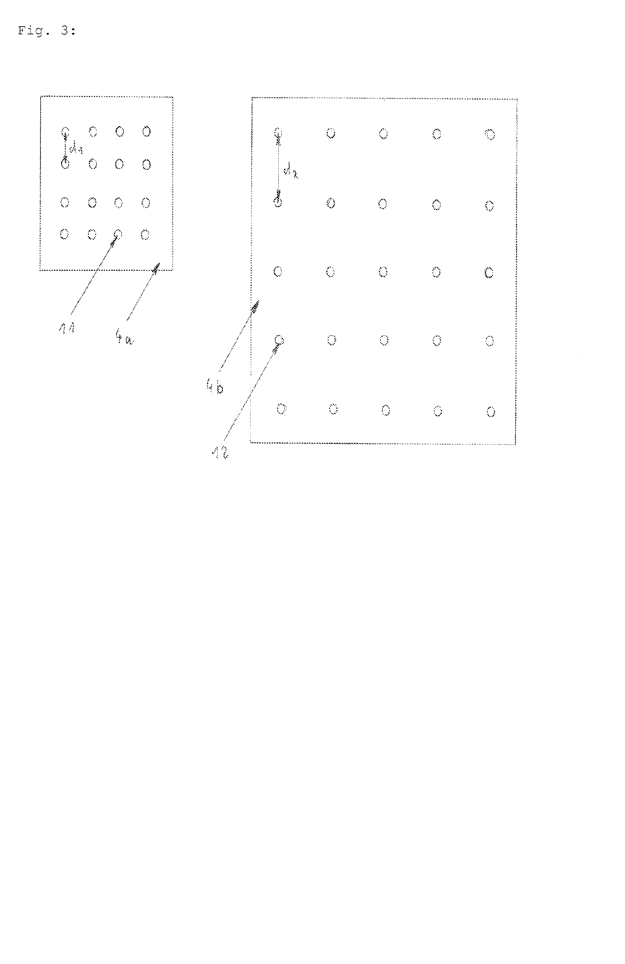

Die Verstelleinheit 3 verstellt die Lage der Führung von Strahler/Dummy 10 so, dass die Führung von Strahler/Dummy 10 an einem ausgewählten ersten Kanal 11 (s. Fig. 3) eines Verteilermoduls 4 ausgerichtet wird. Ein Kanal 11 ist dabei ein Hohlraum, in dem ein Strahler oder ein Dummy 10 geführt werden kann. Die Verstelleinheit 3 fährt bevorzugt über eine miniaturisierte Antriebseinheit 19 eine x-y Matrix erster Kanäle 11 ab. Die Antriebseinheit 19 platziert den Strahler/ Dummy 10 immer vor genau einem ersten Kanal 11 der Matrix.

Auf der Verstelleinheit 3 kann weiter ein Sensor 6c zur Abfrage der Kanalbelegung befestigt sein. Der Sensor ermittelt welche Kanäle in der Anschlussplatte aktuell von Transferschläuchen oder Applikatoren belegt sind.

An die Matrix schließt sich ein Verteilermodul 4 an. Bevorzugt ist die Matrix ein Teil des Verteilermoduls 4, genauer ihre Eingangsseite 4a, wie in Fig. 3 gezeigt. Die Verstelleinheit 3 fährt also die Eingangsseite 4a des Verteilermoduls 4 ab.

In einem Ausführungsbeispiel spreizt das Verteilermodul 4 die Matrix zur Ausgangsseite 4b weiter auf, siehe Figur 3. Der Abstand d₁ zwischen den Kanälen11 ist auf der Eingangsseite 4a kleiner als der Abstand d₂ zwischen den Kanälen12 auf der Ausgangsseite 4b. Das Verteilermodul 4 verfügt dabei über eine gewisse Länge, die es erlaubt, den Stress für den Strahler 10 bei der Aufspreizung zu begrenzen. Die Umlenkung erfolgt hier also mit großen Radien. Im Verteilermodul 4 werden die Abstände zwischen den einzelnen Kanälen der y-x Matrix auf ein Maß gespreizt, dass eine gute Zugänglichkeit für den Anwender ermöglicht.

Jedoch kann alternativ in einem anderen Ausführungsbeispiel auch keine weitere Aufspreizung erfolgen und der Abstand d₁ zwischen den ersten Kanälen 11 kann gleich dem Abstand d₂ zwischen den zweiten Kanälen 12 sein. Dann weist das Verteilermodul 4 nur einen geringen Längenbedarf auf. Ein solches Verteilermodul 4 ist in Fig. 2 gezeigt.

Das Verteilermodul 4 ist mit der Anschlussplatte 5 für eine Applikation bzw. für mehrere Applikatoren verbunden, die sich an das Verteilermodul 4 anschließt, bevorzugt direkt anschließt. An der Anschlussplatte 5 können Applikatoren oder eine Applikation direkt angeschlossen werden. Es können aber auch bei Bedarf wiederum Transferschläuche zu einer Applikation dort angeschlossen werden. Die Anschlussplatte 5 weist zweite Kanäle 12 zur Überführung von Strahler/Dummy 10 in die Applikationen/Applikatoren auf.

Die Anschlussplatte Applikation/Applikatoren 5 kann bevorzugt durch einen Rastmechanismus einfach und schnell gewechselt werden. Das Gerät kann über eine Kodierung erkennen, welche Anschlussplatte Applikation 5 aktuell verwendet wird und welche ersten Kanäle 11 somit tatsächlich zweiten Kanälen 12 entsprechen. Dazu können die Anschlussplatten 5 z.B. mit einem Kodierstecker versehen, der dann in einem Gegenstück (Buchse) am Verteilermodul 4 über eine Kontaktierung eine Auswertung ermöglicht.

Mit der Anschlussplatte Applikation/Applikatoren 5 können dann verschiedene Anschlussvarianten abgebildet werden. Die Anzahl der ersten Kanäle 11 kann der Anzahl der zweiten Kanäle 12 entsprechen oder auch größer sein.

Mit Hilfe von Kupplungen können Applikationen wie flexible Katheter oder Nadeln auch direkt angeschlossen werden. Über die Anzahl der angebotenen zweiten Kanäle 12 ist das System einfach skalierbar. Es werden jeweils die zweiten Kanäle 12 genutzt, die eine gute Zugänglichkeit ermöglichen. Also die zweiten Kanäle 12 mit möglichst großen Abständen untereinander.

Es kann somit mittels der modularen Anschlussplatte 5 erfindungsgemäß die komplette Applikation mit einem Handgriff vom Strahlerweichenmodul 1 getrennt werden.

In einer anderen Variante wird die Ankopplung des Strahlerweichenmoduls 1 an ein Template ermöglicht. Diese Templates werden u.a. auch bei der temporären Brachytherapie des Prostatakarzinoms verwendet. Das Template dient dabei zur Führung sowie zur Fixierung von eingesetzten Nadeln in die matrixförmig angeordneten zweiten Kanäle 12. Das Strahlerweichenmodul 1 wird direkt am Template positioniert und über einen Rastmechanismus mit diesem verbunden. Die im Template geführten Nadeln werden dabei über konisch auslaufende Bohrungen des Verteilermoduls gefangen und dann geführt. Die für Dummy und Strahler 10 erforderliche Führung im Bereich der Aufnahmekanäle kann durch federnde Ausgleichselemente gewährleistet werden.

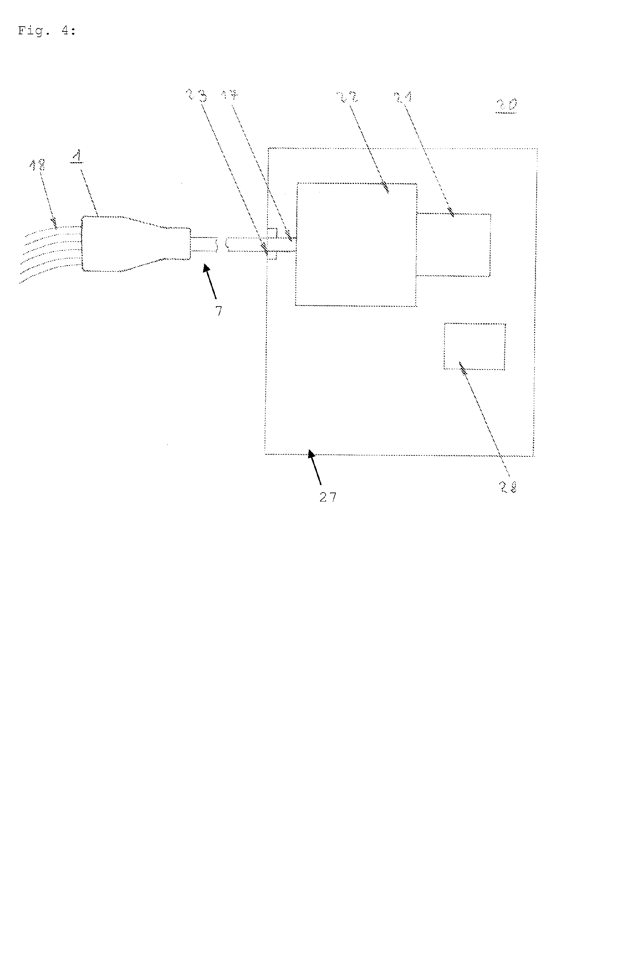

Figur 4 zeigt das erfindungsgemäße Afterloadersystem in einer ersten Ausführungsform. Hier sind Strahlerweichenmodul 1 und Afterloader 20 lediglich über eine einzige Transferverbindung 7 verbunden. Das Strahlerweichenmodul 1 kann dabei über eine z. B. an der Decke oder an einem Behandlungsbett montierte Halterung im Bereich der Applikation gehalten werden. Das Strahlerweichenmodul 1 und seine möglichen Ausbildungen wurden bereits zuvor beschrieben und diese Beschreibung wird hier nicht wiederholt, sondern auf obige Absätze sowie die Beschreibung der Figur 2 verwiesen.

Der Afterloader 20 umfasst dabei Mittel zum Vortrieb von Strahler oder Dummies 21. Es kann auch je ein Mittel zum Vortrieb eines Strahlers und ein Mittel zum Vortrieb eines Dummies vorgesehen sein. Der Afterloader 20 weist ferner eine Abschirmung 22 des Afterloaders 20 zur Vermeidung des Austritts von Strahlung auf. Des Weiteren ist eine Austrittsöffnung 23 vorgesehen, aus der mindestens ein Kanal herausführt, z. B. ein Kanal für einen Strahler oder einen Dummy 10 oder bevorzugt zwei Kanäle, je einer für einen Strahler und einer für einen Dummy 10. Dies ermöglicht, sowohl einen Strahler als auch einen Dummy 10 in die Transferverbindung 7 und den dort enthaltenen Fahrkanal 17/ die Fahrkanäle 17 zu überführen. Ferner kann aus der Austrittsöffnung 23 auch eine Signalleitung 14 und/oder eine Energieversorgungsleitung 15 herausführen. Eine Transferverbindung 7 wird über eine Kupplung mit der Austrittsöffnung 23 verbunden. Die Transferverbindung 7 umfasst mindestens eine Fahrkanal 17, in den ein Strahler/Dummy 10 aufgenommen werden kann. Es können auch zwei Fahrkanäle 17 vorgesehen sein, je einer für einen Strahler 10 und einen Dummy 10. Der Afterloader 20 ist von einem Gehäuse umschlossen.

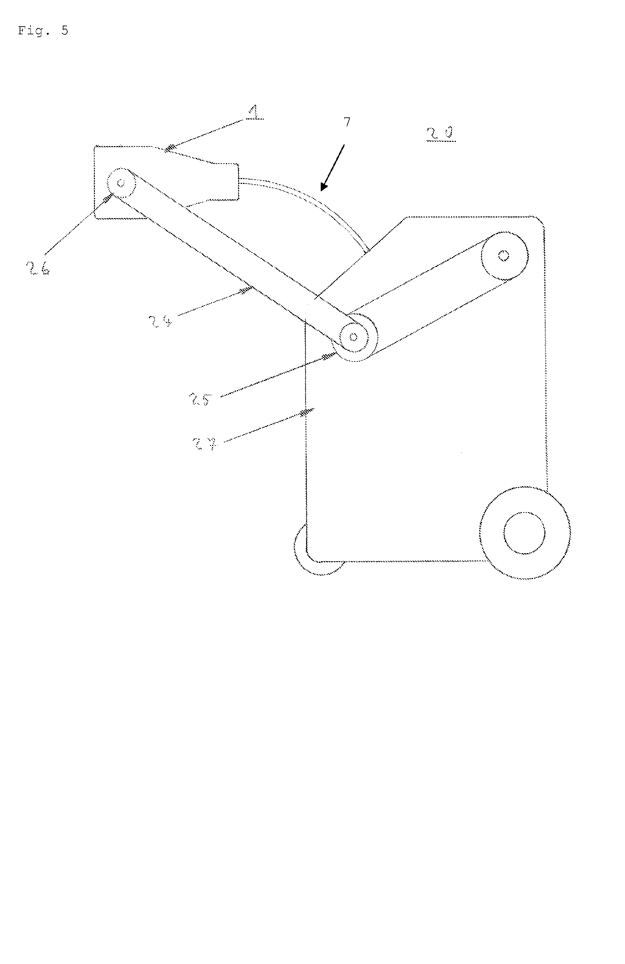

In einem zweiten Ausführungsbeispiel der Figur 5 ist ein Schwenkarm 24 am Gehäuse 27 des Afterloaders 20 angeordnet. Der Schwenkarm 24 verfügt über mindestens ein Gelenk 25. Alternativ kann auch ein ausziehbarer Arm vorgesehen sein. Ferner weist der Schwenkarm 24 an seinem vom Gehäuse 27 getrennten Ende eine Aufnahme bzw. ein Mittel zur Arretierung 26 des Strahlerweichenmoduls 1 am Schwenkarm 24 auf. Das Strahlerweichenmodul 1 kann über das Mittel zur Arretierung 26 am Schwenkarm 24 befestigt werden und über den Schwenkarm 24 zur Applikation verbracht werden, ohne das der Afterloader 20 selbst bewegt werden müsste. Das Mittel zur Arretierung 26 kann Schrauben und Schraublöcher umfassen, aber bevorzugt einfach trennbare Verbindungen wie Clips oder ein Nut/Fugensystem.

Ferner weist ein Afterloader 20 einen Mikroprozessor 28, der sowohl den Vortrieb im Gehäuse 27 der Nachladevorrichtung 20 als auch Daten der Sensoren 6 in dem Strahlerweichenmodul 1 erhält und auswertet sowie mit der Steuerung im Nachladegerät kommuniziert.

Bei einem externen Betrieb der Figur 5 ist das Strahlerweichenmodul 1 über die Transferverbindung 7 mit dem Afterloader 20 verbunden und mittels des Schwenkarms 24 im Bereich der Anwendung positionierbar. Der Schwenkarm 24 ist dabei an dem Afterloader 20 befestigt, verbindet also Nachladegerät 20 und Strahlerweichenmodul 2. Die Transferverbindung 7 kann extern am Schwenkarm 24 geführt werden. Der Schwenkarm 24 steht dann in Kommunikation mit der Austrittsöffnung 23 des Afterloader 20.

### Bezugszeichenliste

- 1: Strahlerweichenmodul
- 2: Gehäuse
- 3: Verstelleinheit
- 4: Verteilermodul
- 4a: Eingangsseite Verteilermodul
- 4b: Ausgangsseite Verteilermodul
- 5: Anschlussplatte Applikationen
- 6: Sensorik
- 6a: Sensor Referenzierung
- 6b: Sensor Wegmessung
- 6c: Sensor Kanalbelegung
- 7: Transferverbindung
- 8: Anschluss Transferverbindung
- 9: Führungskanal
- 10: Strahler oder Dummy
- 11: erste Kanäle des Verteilermoduls
- 12: zweite Kanäle der Anschlussplatte
- 13: Energiequelle
- 14: Energieversorgungsleitung
- 15: Signalleitungen
- 16: Abschirmung Transferverbindung
- 17: Fahrkanal
- 18: Transferschlauch
- 19: Antriebseinheit
- 20: Afterloader
- 21: Mittel zum Vortrieb von Strahler oder Dummies
- 22: Abschirmung Afterloader
- 23: Austrittsöffnung
- 24: Schwenkarm
- 25: Gelenk Schwenkarm
- 26: Aufnahme an der Afterloader bzw. Mittel zur Arretierung
- 27: Gehäuse Afterloader
- 28: Mikroprozessor
- 100: Afterloader des Standes der Technik
- 101: Gehäuse
- 102: Strahlerweiche
- 103: Kanäle
- 104: Transferschläuche

## Patentansprüche

1. Strahlerweichenmodul (1) für einen Brachytherapie-Afterloader umfassend:
eine Aufnahme (8) für eine Transferverbindung (7) eines Strahlers/Dummies (10);
ein Mittel (6a) zur Referenzierung der Position eines Strahlers/Dummies (10);
ein Verteilermodul (4) umfassend eine Eingangsseite (4a) mit einer Mehrzahl von ersten Kanälen (11) und eine Ausgangsseite (4b) mit einer Mehrzahl von zweiten Kanälen (12), wobei jeder erste und jeder zweite Kanal (11, 12) zur Aufnahme eines Strahlers/Dummies (10) ausgebildet ist;
eine Verstelleinheit (3) zur Positionierung eines Strahlers/Dummies (10) vor einem ersten Kanal (11) des Verteilermoduls (4);
einen Führungskanal (9) zur Führung eines Strahlers/Dummies (10) von der Aufnahme (8) zur Verstelleinheit (3), und
ein Mittel (5) zum Anschluss von Applikationen verbunden mit der Ausgangsseite des Verteilermoduls (4).

2. Strahlerweichenmodul (1) nach Anspruch 1, ferner umfassend ein Mittel (6b) zur Messung des Vortriebs eines Strahlers/Dummies (10).

3. Strahlerweichenmodul (1) nach Anspruch 1 oder 2, ferner umfassend eine Antriebseinheit (19) zum Betrieb der Verstelleinheit (3) sowie einen Anschluss an eine externe Energieversorgung.

4. Strahlerweichenmodul (1) nach Anspruch 3, wobei der Anschluss an eine externe Energieversorgung durch die Aufnahme (8) ausgebildet ist, über den der Strahler/Dummy (10) geführt wird.

5. Strahlerweichenmodul (1) nach einem der vorhergehenden Ansprüche, wobei der Führungskanal (9) durch die Transferverbindung (7) selbst ausgebildet ist, die mit der Verstelleinheit (3) verbunden ist.

6. Strahlerweichenmodul (1) nach einem der vorhergehenden Ansprüche, wobei das Mittel (5) zum Anschluss von Applikationen austauschbar ausgebildet ist.

7. Strahlerweichenmodul (1) nach einem der vorhergehenden Ansprüche, wobei die ersten Kanäle (11) und die zweiten Kanäle (12) derart ausgebildet sind, dass zu jedem zweiten Kanal (12) mindestens ein erster Kanal (11) vorhanden ist.

8. Strahlerweichenmodul (1) nach einem der vorhergehenden Ansprüche, wobei das Verteilermodul (4) einen von der Eingangsseite (4a) her wachsenden oder gleichen Querschnitt aufweist.

9. Brachytherapie-Afterloadersystem , umfassend:
- einen Afterloader (20) umfassend:
- ein Gehäuse (27),
- im Inneren des Gehäuses angeordnete Strahler und Dummies (10),
- mindestens ein Mittel zum Vortrieb (21) von Strahler/Dummy (10),
- eine erste Abschirmung (22) zur Strahlungsabschirmung, und
- eine Austrittsöffnung (23) für Strahler und/oder Dummy (10);
- ein Strahlerweichenmodul (1) gemäß einem der vorhergehenden Ansprüche 1-8; und
- eine Transferverbindung (7) zur Verbindung von Nachladevorrichtung (20) und Strahlerweichenmodul (1), wobei die Transferverbindung ausgebildet ist, einen Strahler/Dummy (10) zu führen.

10. Brachytherapie- Afterloadersystem nach Anspruch 9, wobei das Strahlerweichenmodul (1) vom Gehäuse (27) der Nachladevorrichtung (20) räumlich getrennt ausgebildet ist und mit der Nachladevorrichtung (20) verbindbar ist.

11. Brachytherapie- Afterloadersystem nach Anspruch 9 oder 10, wobei die Transferverbindung (7) über die Austrittsöffnung (23) für Strahler und/oder Dummies (10) mit der Nachladevorrichtung (20) verbunden ist und über die Aufnahme (8) für die Transferverbindung (7) mit dem Strahlerweichenmodul (1) verbunden ist.

12. Brachytherapie- Afterloadersystem nach einem der Ansprüche 9 bis 11, wobei die Transferverbindung (7) eine Energieversorgungsleitung (14) und/oder Signalleitungen (15) und/oder eine zweite Abschirmung (16) zur Strahlungsabschirmung umfasst.

13. Brachytherapie- Afterloadersystem nach einem der vorhergehenden Ansprüche 9 bis 12, wobei die Austrittsöffnung (23) sowohl eine Austrittsöffnung für einen Strahler (10) wie auch eine Austrittsöffnung für einen Dummy (10) umfasst.

14. Brachytherapie- Afterloadersystem einem der vorhergehenden Ansprüche 9 bis 13, wobei ein Schwenkarm (24) am Gehäuse (27) der Nachladevorrichtung (20) angeordnet ist und an dem das Strahlerweichenmodul (1) befestigbar ist.

15. Brachytherapie- Afterloadersystem nach einem der vorhergehenden Ansprüche 9 bis 14, ferner umfassend einen Mikroprozessor (28), der das mindestens eine Mittel zum Vortrieb (21) von Strahler/Dummy (10) steuert und der über die Signalleitungen (15) in der Transferverbindung (7) Daten des Mittels (6) zur Referenzierung und/oder der Messung des Vortriebs aus dem Strahlerweichenmodul (1) erhält.
